# EUROPEAN PATENT APPLICATION

(11) **EP 4 342 460 A1**
(43) Date of publication of application: **27.03.2024**
(21) Application number: 22196901.7
(22) Date of filing: 21.09.2022
(51) Int. Cl.: A61K 9/51

(54) **LIPID NANOPARTICLE WITH NUCLEIC ACID CARGO**

(71) Applicant: NovoArc GmbH, 1060 Wien (AT)
(72) Inventor: WURM, David, 7350 Oberpullendorf (AT); QUEHENBERGER, Julian, 1120 Wien (AT); SPADIUT, Oliver, 1070 Wien (AT); SEDLMAYR, Viktor, 1160 Wien (AT)
(74) Representative: SONN Patentanwälte GmbH & Co KG

(57) **Abstract**

Disclosed is a lipid nanoparticle (LNP) encapsulating a nucleic acid cargo preferably comprising messenger ribonucleic acid (mRNA). The LNP comprises at least a cationic lipid fraction, and a stabilizer fraction. The stabilizer fraction preferably comprises at least one polyethylenglycol (PEG) lipid. Furthermore, the LNP comprises at least one glycerol dialkyl glycerol tetraether (GDGT) lipid, as obtained e.g. from archaea of the genus *Sulfolobus,* optionally among other ether lipids. Also disclosed is a pharmaceutical composition comprising the LNP, such as an mRNA vaccine.

## Description

The field of the present invention relates to lipid nanoparticles (LNPs) with nucleic acid cargo, in particular as used in messenger ribonucleic acid (mRNA) vaccines.

LNPs have recently come into focus because LNP-based mRNA vaccines against SARS-CoV-2 (primarily elasomeran marketed under Spikevax^{®} by Moderna Inc., and tozinameran marketed under Comirnaty^{®} by Biontech SE/Pfizer Inc) were administered to hundreds of millions of individuals. LNPs as delivery systems for RNA-based vaccines in general are reviewed in Aldosari et al., 2021. LNPs for mRNA delivery are discussed in detail also in Hou et al, 2021.

In general, LNPs with nucleic acid cargo (or payload) comprise a lipid layer as well as microdomains of lipid and encapsulated nucleic acid. They have a median diameter between 10 nm to 1000 nm (e.g. as determined by dynamic light scattering, DLS) and may adopt e.g. a spherical or polyhedral shape. They may be multilamellar, dependent on their specific lipid composition. The LNPs comprise cationic lipids (in particular lipids which are protonated at low pH, i.e. when in an endosome, also called ionizable lipids). Typically, the LNPs further comprise stabilizers such as polyethylenglycol (PEG) lipids which decrease LNP aggregation. In addition, LNPs usually comprise other types of lipid (often termed "helper lipids"), such as phosphatidylcholines or phosphatidylethanolamines, to improve properties such as delivery efficacy, tolerability or biodistribution. Finally, LNPs may contain cholesterol or other sterols to modulate membrane integrity and rigidity.

LNPs are for instance also disclosed in US patents US 7,404,969, US 8,058,069, US 9,364,435 and US 9,404,127.

WO 2017/099823 A1 discloses an accelerated-blood-clearance-insensitive LNP, comprising a cationic lipid, a polyethylene glycol (PEG)-lipid, a sterol, and a helper lipid, wherein the helper lipid does not comprise a phosphatidyl choline.

WO 2020/061284 A1 also concerns LNPs with PEG lipids.

WO 2020/219941 A1 discloses further LNPs and formulations containing LNPs.

WO 2021/123332 A1 relates to cationic lipids and to LNPs comprising said cationic lipids useful for the delivery of nucleic acids into living cells.

Despite the recent advances in the field, there is still a need for improved LNPs, in particular with regard to storage stability and/or transformation (transfection) efficiency. For instance, the LNP-based SARS-CoV-2 vaccine Comirnaty^{®} generally has to be stored at -90°C to -60°C (Summary of product characteristics, version of 13 September 2022, EMEA/H/C/005735 - 11/0143, European Medicines Agency - EMA). Furthermore, an increase in transformation efficiency would allow for lower doses, thereby decreasing potential side effects.

It is thus an object of the present invention to provide an improved LNP with nucleic acid cargo (such as mRNA cargo), in particular an LNP that has increased transformation efficiency and/or higher storage stability, especially at non-refrigerated temperatures (e.g., room temperature).

The present invention provides an LNP encapsulating a nucleic acid cargo. The LNP comprises at least a cationic lipid fraction (which preferably comprises at least one ionizable lipid) and a stabilizer fraction (preferably comprising PEG lipids). The LNP comprises at least one glycerol dialkyl glycerol tetraether (GDGT) lipid.

The present invention also provides a pharmaceutical composition (in particular a vaccine) comprising the LNP. This pharmaceutical composition typically comprises further excipients. It is preferably for use in prevention or treatment of a disease or condition in a (human) patient, in particular as a vaccine to prevent (or ameliorate) a disease such as an infectious disease or as a cancer vaccine.

In the course of the present invention, experiments were conducted to improve LNPs known in the prior art (such as the LNPs used in the Comirnaty^{®} mRNA vaccine). Surprisingly, it turned out that addition of GDGT lipids to the LNP formulation increased both transformation efficiency in target cells and storage stability of the LNPs.

Importantly, LNPs (as described in more detail above) are distinct from other lipid-containing delivery vehicles such as liposomes, lipo(poly)plexes or archaeosomes and present unique advantages but also challenges, especially in the context of packaging mRNA. For instance, Midoux & Pichon, 2014, reviews various lipid-based mRNA vaccine delivery systems, and distinguishes between lipoplexes, lipopolyplexes, LNPs and cationic nanoemulsions. More broadly, drug delivery for RNA therapeutics (such as small interfering RNAs and mRNAs) is enabled by lipid-based vehicles such as micelles, liposomes and LNPs (Paunovska et al, 2022).

In contrast to LNPs, liposomes are spherical lipid bilayer vesicles (lipid esters) surrounding an aqueous space. They are carriers for the administration of drugs, vaccines, genes, proteins, small molecules, antibiotics and nutrients. Liposomes are made of phospholipids, mainly phosphatidylcholine, and cholesterol, but may also include other lipids, like phosphatidylethanolamine. Liposomes are generated by a large number of different methods (reviewed e.g. by van Hoogevest, 2017; Szoka et al., 1980), for example by dispersing phospholipids in aqueous medium, e.g. using mechanical treatment (e.g. in a homogenizer, preferably by high pressure homogenization) or sonication. They vary between 0.02 to 10 µm in diameter.

Archaeosomes represent a special class of liposomes based on membrane lipids isolated from archaea. Archaeosomes are made of lipid ethers, namely diether structures (such as archaeols) and tetraether structures (e.g. GDGTs, such as caldarchaeols); see e.g. Kaur et al., 2016; Patel et al., 1999. Diether structures are typically composed of a glycerol moiety carrying two phytanyl chains (20-40 carbons in length) on the sn-2,3 positions. Tetraether structures typically carry two di-phytanyl chains linked to two glycerol residues in either an antiparallel manner (caldarchaeol) or a parallel manner (iso-caldarchaeol). Furthermore, one or several cyclopentane rings may occur.

As used herein, "caldarchaeol" refers to the whole group of isoprenoid GDGT lipids with 0 up to 8 cyclopentane moieties. In particular, the nomenclature suggested by Schouten et al., 2013, is used herein: "GDGT-x", where x denotes the number of cyclopentane moieties, i.e. GDGT-0, GDGT-1, GDGT-2, GDGT-3, GDGT-4, GDGT-5, GDGT-6, GDGT-7 and GDGT-8. All of these belong to the group of caldarchaeols.

Depending on the composition (e.g. amount of archaeols vs. caldarchaeols), the lipid layer of archaeosomes is a mono- or a bi-layer or a mixture thereof.

WO 2020/187526 A1 discloses archaeosomes comprising archaeal lipids from a *Sulfolobus* cell culture, mainly intended for oral acute or oral retard delivery.

Vishakarma et al, 2019, reviews various lipid-based carriers for lymphatic transportation, among them liposomes and archaeosomes.

Further in relation to archaeosomes, Daswani et al, 2021, discloses that the polar lipid fraction E from *Sulfolobus acidocaldarius* can be used as liposomal drug stabilizing agents to reduce the leakage of the antivascular drug combretastatin a4 disodium phosphate from tetraether/diester hybrid archaeosomes.

In contrast to archaeosomes, LNPs contain cationic lipids (in particular ionizable lipids).

In a preferred embodiment of the present invention, the cationic lipid is an ionizable lipid (i.e. a lipid which carries an overall positive charge at endosomal pH, e.g. a pH between 5.0-6.5, such as pH 5.0, 5.5, 6 or 6.5, but is neutral at a higher pH such as pH 7.0).

In another preferred embodiment, the cationic lipid fraction comprises at least one cationic lipid (such as an ionizable lipid) selected from the group consisting of [(4-hydroxybutyl)azanediyl]di(hexane-6,1-diyl) bis(2-hexyldecanoate) (ALC-0315), heptadecan-9-yl 8-{(2-hydroxyethyl)[6-oxo-6-(undecyloxy)hexyl]amino}octanoate (SM-102),3-(didodecylamino)-N1,N1,4-tridodecyl-1-piperazineethanamine (KL10), N1-[2-(didodecylamino)ethyl] N1,N4,N4-tridodecyl-1,4-piperazinediethanamine (KL22), 14,25-ditridecyl-15,18,21,24-tetraaza-octatriacontane (KL25), 1,2-dilinoleyloxy-N,N-dimethylaminopropane (DLin-DMA), 2,2-dilinoleyl-4-dimethylaminomethyl-[1,3]-dioxolane (DLin-K-DMA), heptatriaconta-6,9,28,31-tetraen-19-yl-4-(dimethylamino)butanoate (DLin-MC3-DMA), 2,2-dilinoleyl-4-(2 dimethylaminoethyl)-[1,3]-dioxolane (DLin-KC2-DMA), 1,2-dioleyloxy-N,N-dimethylaminopropane (DODMA), 2-({8 [(3β)-cholest-5-en-3-yloxy]octyl}oxy) N,N dimethyl-3-[(9Z,12Z)-octadeca-9,12-dien-1-yloxy]propan-1-amine (Octyl-CLinDMA), (2R)-2-({8-[(3β)-cholest-5-en-3-yloxy]octyl}oxy)-N,N-dimethyl-3-[(9Z,12Z)-octadeca-9,12-dien-1-yloxy]propan-1-amine (Octyl-CLinDMA (2R)), (2S) 2-({8-[(3β)-cholest-5-en-3-yloxy]octyl}oxy)-N,N-dimethyl-3-[(9Z,12Z)-octadeca-9,12-dien-1-yloxy]propan-1-amine (Octyl-CLinDMA (2S)) and mixtures thereof. Alternatively, or in addition thereto, the cationic lipid is preferably selected from the group consisting of (20Z,23Z)-N,N-dimethylnonacosa-20,23-dien-10-amine, (17Z,20Z)-N,N-dimemylhexacosa-17,20-dien-9-amine, (1Z,19Z)-N5N-dimethylpentacosa-16, 19-dien-8-amine, (13Z,16Z)-N,N-dimethyldocosa-13,16-dien-5-amine, (12Z,15Z)-N,N dimethylhenicosa-12,15-dien-4-amine, (14Z,17Z)-N,N-dimethyltricosa-14,17-dien-6-amine, (15Z,18Z)-N,N-dimethyltetracosa-15,18-dien-7-amine, (18Z,21Z)-N,N-dimethylheptacosa-18,21-dien-10-amine, (15Z,18Z)-N,N-dimethyltetracosa-15,18-dien-5-amine, (14Z,17Z)-N,N-dimethyltricosa-14,17-dien-4-amine, (19Z,22Z)-N,N-dimeihyloctacosa-19,22-dien-9-amine, (18Z,21Z)-N,N-dimethylheptacosa-18,21-dien-8-amine, (17Z,20Z)-N,N-dimethylhexacosa-17,20-dien-7-amine, (16Z,19Z)-N,N-dimethylpentacosa-16,19-dien-6-amine, (22Z,25Z)-N,N-dimethylhentriaconta-22,25-dien-10-amine, (21Z,24Z)-N,N-dimethyltriaconta-21,24-dien-9-amine, (18Z)-N,N-dimetylheptacos-18-en-10-amine, (17Z)-N,N-dimethylhexacos-17-en-9-amine, (19Z,22Z)-N,N-dimethyloctacosa-19,22-dien-7-amine, N,N-dimethylheptacosan-10-amine, (20Z,23Z)-N-ethyl-N-methylnonacosa-20,23-dien-10-amine, 1-[(11Z,14Z)-1-nonylicosa-11,14-dien-1-yl]pyrrolidine, (20Z)-N,N-dimethylheptacos-20-en-10-amine, (15Z)-N,N-dimethyl eptacos-15-en-10-amine, (14Z)-N,N-dimethylnonacos-14-en-10-amine, (17Z)-N,N-dimethylnonacos-17-en-10-amine, (24Z)-N,N-dimethyltritriacont-24-en-10-amine, (20Z)-N,N-dimethylnonacos-20-en-10-amine, (22Z)-N,N-dimethylhentriacont-22-en-10-amine, (16Z)-N,N-dimethylpentacos-16-en-8-amine, (12Z,15Z)-N,N-dimethyl-2-nonylhenicosa-12,15-dien-1-amine, (13Z,16Z)-N,N-dimethyl-3-nonyldocosa-13,16-dien-1-amine, N,N-dimethyl-1-[(1S,2R)-2-octylcyclopropyl] eptadecan-8-amine, 1-[(1S,2R)-2-hexylcyclopropyl]-N,N-dimethylnonadecan-10-amine, N,N-dimethyl-1-[(1S,2R)-2-octylcyclopropyl]nonadecan-10-amine, N,N-dimethyl-21-[(1S,2R)-2-octylcyclopropyl]henicosan-10-amine,N,N-dimethyl-1-[(1S,2S)-2-1[(1R,2R)-2-pentylcyciopropyl]methyl}cyclopropyl]nonadecan-10-amine,N,N-dimethyl-1-[(1S,2R)-2-octylcyclopropyl]hexadecan-8-amine, N,N-dimethyl-[(1R,2S)-2undecyIcyclopropyl]tetradecan-5-amine, N,N-dimethyl-3-{7-[(1S,2R)-2-octylcyclopropyl]heptyl} dodecan-1-amine, 1-[(1R,2S)-2-heptylcyclopropyl]-N,N-dimethyloctadecan-9-amine, 1-[(1S,2R)-2-decylcyclopropyl]-N,N-dimethylpentadecan-6-amine, N,N-dimethyl-1-[(1S,2R)-2-octylcyclopropyl]pentadecan-8-amine, R-N,N-dimethyl-1-[(9Z,12Z)-octadeca-9,12-dien-1-yloxy]-3-(octyloxy)propan-2-amine, S-N,N-dimethyl-1-[(9Z,12Z)-octadeca-9,12-dien-1-yloxy]-3-(octyloxy)propan-2-amine, 1-{2-[(9Z,12Z)-octadeca-9,12-dien-1-yloxy]-1-[(octyloxy)methyl]ethyl}pyrrolidine, (2 S)-N,N-dimethyl-1-[(9Z,12Z)-octadeca-9,12-dien-1-yloxy]-3-[(5Z)-oct-5-en-1-yloxy]propan-2-amine, 1-{2-[(9Z,12Z)-octadeca-9,12-dien-1-yloxy]-1-[(octyloxy)methyl]ethyl}azetidine, (2S)-1-(hexyloxy)-N,N-dimethyl-3-[(9Z,12Z)-octadeca-9,12-dien-1-yloxy]propan-2-amine, (2S)-1-(heptyloxy)-N,N-dimethyl-3-[(9Z,12Z)-octadeca-9,12-dien-1-yloxy]propan-2-amine, N,N-dimethyl-1-(nonyloxy)-3-[(9Z,12Z)-octadeca-9,12-dien-1-yloxy]propan-2-amine, N,N-dimethyl-1-[(9Z)-octadec-9-en-1-yloxy]-3-(octyloxy)propan-2-amine; (2S)-N,N-dimethyl-1-[(6Z,9Z,12Z)-octadeca-6,9,12-trien-1-yloxy]-3-(octyloxy)propan-2-amine, (2S)-1-[(11Z,14Z)-icosa-11,14-dien-1-yloxy]-N,N-dimethyl-3-(pentyloxy)propan-2-amine, (2S)-1-(hexyloxy)-3-[(11Z,14Z)-icosa-11,14-dien-1-yloxy]-N,N-dimethylpropan-2-amine, 1-[(11Z,14Z)-icosa-11,14-dien-1-yloxy]-N,N-dimethyl-3-(octyloxy)propan-2-amine, 1-[(13Z,16Z)-docosa-13,16-dien-1-yloxy]-N,N-dimethyl-3-(octyloxy)propan-2-amine, (2S)-1-[(13Z,16Z)-docosa-13,16-dien-1-yloxy]-3-(hexyloxy)-N,N-dimethylpropan-2-amine, (2S)-1-[(13Z)-docos-13-en-1-yloxy]-3-(hexyloxy)-N,N-dimethylpropan-2-amine, 1-[(13Z)-docos-13-en-1-yloxy]-N,N-dimethyl-3-(octyloxy)propan-2-amine, 1-[(9Z)-hexadec-9-en-1-yloxy]-N,N-dimethyl-3-(octyloxy)propan-2-amine, (2R)-N,N-dimethyl-H(1-metoyloctyl)oxy]-3-[(9Z,12Z)-octadeca-9,12-dien-1-yloxy]propan-2-amine, (2R)-1-[(3,7-dimethyloctyl)oxy]-N,N-dimethyl-3-[(9Z,12Z)-octadeca-9,12-dien-1-yloxy]propan-2-amine, N,N-dimethyl-1-(octyloxy)-3-({8-[(1S,2S)-2-[(1R,2R)-2-pentylcyclopropyl]methyl}cyclopropyl]octyl}oxy)propan-2-amine, N,N-dimethyl-1-{[8-(2-oclylcyclopropyl)octyl]oxy}-3-(octyloxy)propan-2-amine, and (11E,20Z,23Z)- N,N-dimethylnonacosa-11,20,2-trien-10-amine, and pharmaceutically acceptable salts and stereoisomers thereof, and mixtures thereof.

Further suitable cationic lipids (in particular ionizable lipids) are disclosed e.g. in Hou et al, 2021, US patents US 7,404,969, US 8,058,069, US 9,364,435 and US 9,404,127, WO 2017/099823 A1, WO 2020/061284 A1, WO 2020/219941 A1 and WO 2021/123332 A1. These documents are included herein by reference in their entirety.

Even further suitable cationic lipids are disclosed e.g. in WO 2017/049245 A1, WO 2017/112865 A1, WO 2012/040184, WO 2011/153120 A1, WO 2011/149733 A1, WO 2011/090965 A1, WO 2011/043913 A1, WO 2011/022460 A1, WO 2012/061259 A1, WO 2012/054365 A1, WO 2012/044638 A1, WO 2010/080724 A1, WO 2010/21865 A1, WO 2008/103276 A1, WO 2013/086373 A1 and WO 2013/086354 A1, US patent nos. 7,893,302, 7,404,969, 8,283,333, and 8,466,122 and US patent publication no. US20100036115, US20120202871, US20130064894, US20130129785, US20130150625, US20130178541, and US20130225836. These documents are incorporated herein by reference in their entirety.

The stabilizer fraction of the inventive LNP is typically suitable for achieving one or more of the following: decreasing LNP aggregation, increasing average particle size or hydrodynamic radius, increasing the half-life of the LNP *in vivo* (e.g. in a human, in particular in blood circulation) and modifying zeta potential.

According to a particular preference, the stabilizer fraction comprises at least one PEG lipid. Suitable PEG lipids are for instance 2-[(polyethylene glycol)-2000]-N,N-ditetradecylacetamide (ALC-0159), pegylated diacylglycerol lipid (PEG-DAG), a pegylated ceramide lipid (PEG-Cer), a pegylated phosphatidylethanoloamine lipid (PEG-PE), a pegylated succinate diacylglycerol lipid (PEG-S-DAG), a pegylated dialkoxypropylcarbamate lipid, 1 ,2-dimyristoyl-rac-glycero-3-methoxypolyethylene glycol ("PEG-DMG" or "DMG-PEG"), in particular PEG2000-DMG, 1,2-dicapryl-rac-glycero-3-methylpolyoxyethylene glycol (Ci₀-diacylglycerol PEG), N-octanoyl-sphingosine-1-{succinyl[methoxy(polyethylene glycol)2000]} (comprising N-octanoyl-D-erythro-sphingosine (d18:1/8:0), also named PEG-Ceramide8), or a PEG lipid as disclosed in WO 2018/126084 A1, WO 2020/093061 A1, or WO 2020/219941 A1 (all three references are incorporated by reference in their entirety); and any combination thereof.

Further suitable PEG lipids are e.g. disclosed in Hou et al, 2021, WO 2017/099823, WO 2020/061284 A1, WO 2020/219941 A1 and WO 2021/123332 A1. All of these documents are incorporated by reference herein in their entirety.

Alternatively, or in addition thereto, the stabilizer fraction may comprise at least one non-PEG moiety such as an XTEN peptide that may or may not be conjugated to a lipid. The XTEN peptide is capable of forming a hydrated shell around the LNP due to its hydrophilic nature. It further serves to increase the half-life of the LNP, compared to an LNP lacking (or free of) a stabilizer fraction. XTEN amino acid sequences are known in the art, including for example those reported in U.S. patent no. 9,062,299 (incorporated herein by reference in its entirety). Alternatively, or in addition thereto, in some embodiments, the stabilizer fraction may comprise non-PEG moiety such as a PAS peptide (that may or may not be conjugated to a lipid). A PAS peptide is a peptide comprising primarily if not exclusively proline, alanine and serine. Like PEG and XTEN peptides, the PAS peptide is capable of forming a hydrated shell around the LNP. It too serves to increase the half-life of an LNP, compared to an LNP lacking (or free of) a stabilizer fraction. PAS amino acid sequences are known in the art, including for example, those reported in WO 2008/155134 A1 (incorporated herein by reference in its entirety).

GDGT lipids (also called "GDGTs" herein) were discovered as membrane lipids of extremophilic archaea but more recently were also observed as membrane components of some bacteria (cf. Schouten et al, 2013). Many phylogenetic groups within the Archaea synthesize GDGTs. They form a monolayer instead of a bilayer in the cell membrane. In the course of the present invention, it turned out that GDGT lipids are exceptionally well suited to improve the properties (such as storage stability and transformation efficiency) of LNPs with nucleic acid cargo.

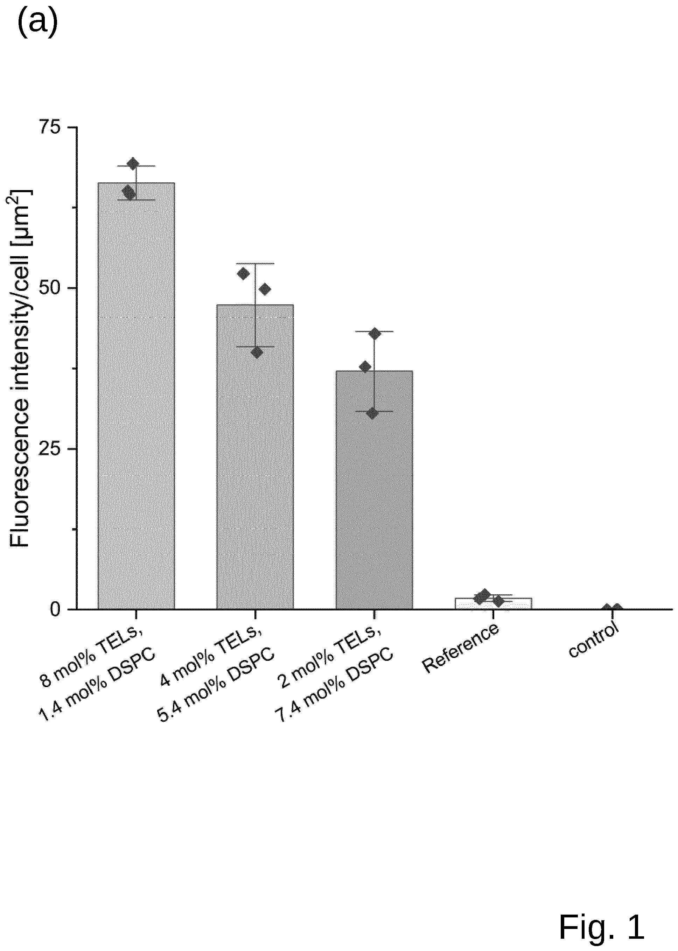

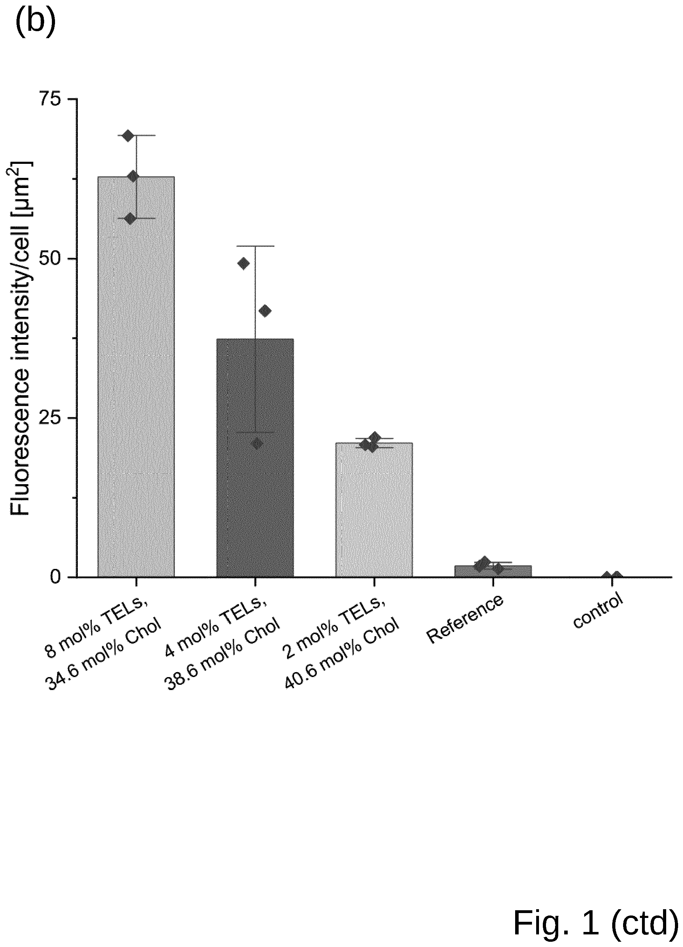

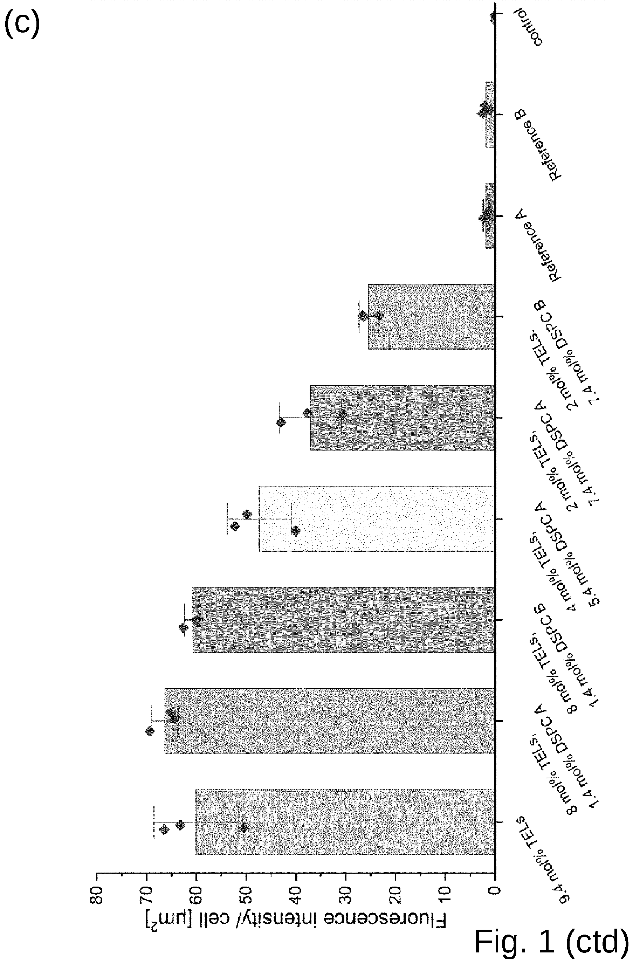

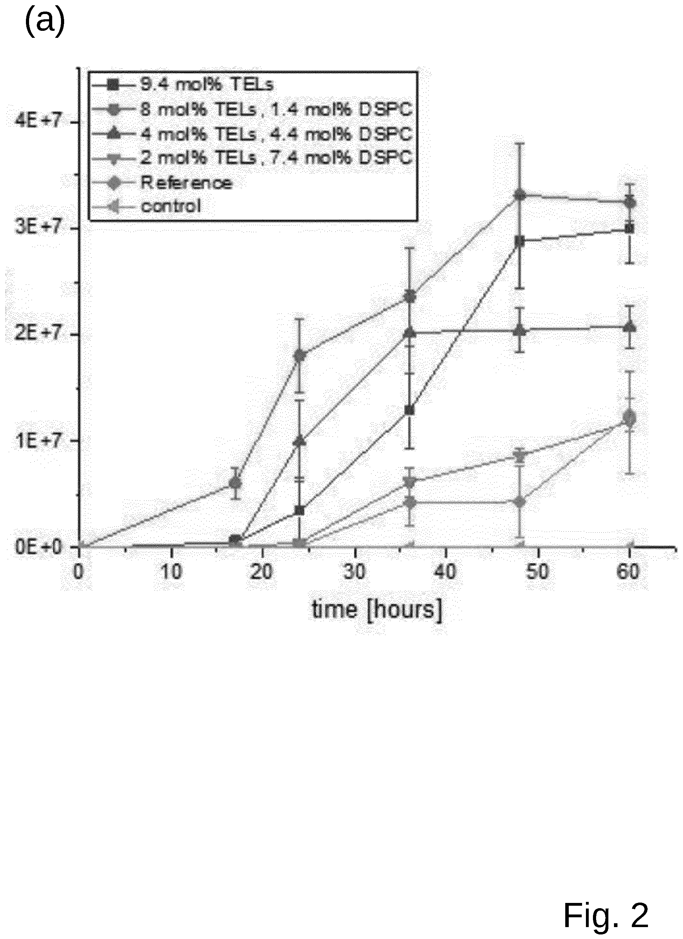

Therefore, as mentioned above, the inventive LNP comprises at least one GDGT lipid. This GDGT lipid may e.g. be an isoprenoid GDGT lipid such as a GDGT-0, a GDGT-1, a GDGT-2, a GDGT-3, a GDGT-4, a GDGT-5, a GDGT-6, a GDGT-7 and a GDGT-8, or crenarchaeol (unsubstituted, cf. Schouten et al, 2013, Fig. 1, or substituted), or a branched GDGT such as GDGT-I, GDGT-II or GDGT-III, or any mixture thereof. In particular, the GDGT lipid may comprise any of the GDGTs disclosed in Schouten et al, 2013 (in particular Fig. 1), Kaur et al, 2016 (in particular Fig. 2, Fig. 3 and Fig. 4), and WO 2020/187526 Al (all of which are incorporated herein by reference in their entirety). The GDGTs may be substituted (e.g. with hexose moieties or phosphatidylinositol moieties) or unsubstituted.

Caldarchaeols (as e.g. obtained from *Sulfolobus*) turned out to be particularly suitable for the present invention, increasing both stability and transformation efficiency of LNPs (see also Examples and Figures). According to a preferred embodiment, at least one GDGT lipid (of the ether lipid fraction of the LNP) thus comprises at least one caldarchaeol, preferably selected from the group consisting of unsubstituted caldarchaeol, phosphatidylinositol (PI)-caldarchaeol, dihexose(2Hex)-caldarchaeol and 2Hex-PI-caldarchaeol (in particular as disclosed in WO 2020/187526 Al, Fig. 4), hexose(Hex)-caldarchaeol and sulfono-trihexose(3Hex)-caldarchaeol, sulfono-3Hex-PI-caldarchaeol, and any mixture thereof.

An especially preferred Hex-caldarchaeol is:

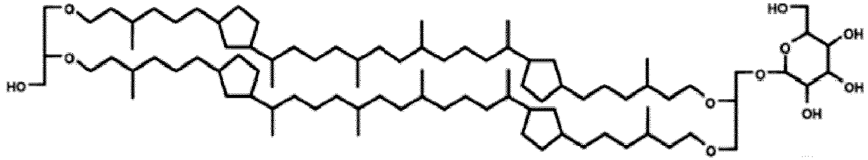

An especially preferred sulfono-3Hex-PI-caldarchaeol is:

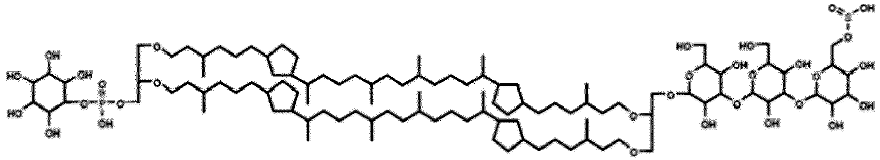

The inventive LNPs are particularly suitable for RNA cargo. Accordingly, in a preferred embodiment, the nucleic acid cargo comprises at least one (therapeutic) RNA. Examples of suitable RNA payloads are for instance disclosed in Paunovska et al, 2022 (in particular Fig. 1). In embodiments, the cargo may be a small interfering RNA (siRNA), an antisense oligonucleotide, an adenosine deaminase acting on RNA (ADAR) oligonucleotide or an mRNA.

The RNA may be chemically modified, e.g. to improve its chemical stability. For instance, it may comprise nucleoside analogs such as analogs having chemically modified bases or sugars, and backbone modifications. In some embodiments, the RNA may comprise nucleoside analogs (e.g, 2- aminoadenosine, 2-thiothymidine, inosine, pyrrolo-pyrimidine, 3-methyl adenosine, 5- methylcytidine, 2-aminoadenosine, C5-bromouridine, C5-fluorouridine, C5-iodouridine, C5- propynyl-uridine, C5-propynyl-cytidine, C5-methylcytidine, 2-aminoadenosine, 7-deazaadenosine, 7-deazaguanosine, 8-oxoadenosine, 8-oxoguanosine, 0(6)-methylguanine, and 2-thiocytidine); chemically modified bases; biologically modified bases (e.g, methylated bases); intercalated bases; modified sugars (e.g, 2'-fluororibose, ribose, 2'-deoxyribose, arabinose, and hexose); and/or modified phosphate groups ( e.g ., phosphorothioates and 5'-N- phosphoramidite linkages). Further modifications are known to the person skilled in the art. Suitable modifications are e.g. disclosed in WO 2017/099823 A1 and WO 2020/061284 A1 (each incorporated by reference in there entirety).

In the course of the present invention, it was found that GDPD lipids improve LNP properties in particular for mRNA payload. According to an especially preferred embodiment, the nucleic acid cargo of the LNP hence comprises an mRNA. The mRNA may e.g. be a therapeutic mRNA or an mRNA encoding a vaccine antigen. The mRNA may be codon-optimized. Examples of suitable mRNAs are for instance given in WO 2017/099823 A1 and WO 2020/061284 A1 (each incorporated by reference in their entirety), in particular paragraphs [00178]-[00180] of the latter.

According to a further preferred embodiment, the LNP comprises at least one further ether lipid, preferably a diether lipid (in particular an archaeol). In particular, the diether lipid lipid may comprise any of the diether lipid disclosed in Kaur et al, 2016 and WO 2020/187526 Al (all of which are incorporated herein by reference in their entirety). The diether lipid (in particular the archaeol) may be substituted (e.g. with hexose moieties or phosphatidylinositol moieties) or unsubstituted. A particularly preferred substituted archaeol is phosphatidyl inositol-archaeol (PI-Arc).

According to another preference, the LNP comprises an ether lipid fraction comprising at least one GDGT lipid (especially "the at least one GDGT lipid" as disclosed above, in particular a caldarchaeol) and preferably at least one further ether lipid (in particular the diether lipid as disclosed above).

It is particularly preferred when the ether lipid fraction comprises ether lipids obtainable by extraction from an archaeal culture, preferably a *Sulfolobus* culture, more preferably a *Sulfolobus acidocaldarius* culture. Suitable growth conditions and extraction methods are e.g. disclosed in WO 2020/187526 A1 (incorporated herein by reference in its entirety). In particular, the entire ether lipid fraction is obtainable by extraction from said culture.

In embodiments, the archaeal culture may e.g. also be a culture of *S. acidocaldarius, M. hungatei, M. voltae, M. concilii, M. smithii, M. espanolae, T. acidophilum, M. mazei, M. espanole, T. acidophilum, H. salinarum, M. smithii, M. stadtmanae, H. halobium, H. morrhuae, M. jannaschii, S. islandicus, S. solfataricus, S. shibatae, S. tokodaii, S. Metallicus, M. sedula, H. hispanica,* or *H.volcanii,* or mixtures (co-cultures) thereof.

By way of example, total archaeal lipids may be extracted from e.g. lyophilized or spray-dried biomass by organic solvent extraction using chloroform/methanol/water. Then, the polar and the neutral lipids may be separated by precipitation using acetone. The resulting lipid extracts may be used directly for the preparation of the ether lipid fraction used for LNP production or may be further purified by chromatography to isolate ether lipids of a particular class to be used for LNP production.

Methods for preparing lipids from archaea or for cultivating archaea are also disclosed e.g. in US 2017/0152533 A1, US 6,316,260 B1, EP 1 999 137 B1, EP 0 883 624 B1, EP 2 109 459 B1, Siliakus et al., 2017, WO 2020/187526 A1, Jain et al., 2014; each incorporated herein by reference in its entirety.

The LNPs themselves may be produced by microfluidic mixing of the LNP components (fractions), including the GDGT lipids (e.g. present in isolated form or in an ether lipid fraction comprising several ether lipids). Suitable LNP production methods are e.g. disclosed in WO 2017/099823 A1, WO 2020/061284 A1, and WO 2021/123332 A1; each incorporated herein by reference in its entirety. Further LNP production methods are available to the person skilled in the art.

According to another preferred embodiment, the LNP further comprises a sterol lipid fraction. Incorporation of sterol lipids in the LNP mitigates aggregation of other lipids in the LNP. Sterol lipids are preferably selected from the group consisting of cholesterol, fecosterol, sitosterol, ergosterol, campesterol, stigmasterol, brassicasterol, phytosterols and mixtures thereof. According to a preferable definition, "sterol lipids" comprise the entire subgroup of steroids which consists of steroid alcohols.

In particular, the sterol lipid fraction comprises cholesterol.

According to yet another preferred embodiment, the LNP further comprises a helper lipid fraction. Helper lipids useful in the present invention comprise (preferably consist of) non-cationic lipids. In particular, the helper lipid may be a phospholipid.

Preferably, the helper lipid is selected from the group consisting of distearoylphosphatidylcholine (DSPC), dioleoylphosphatidylethanolamine (DOPE), Dipalmitoylphosphatidylcholine (DOPC), phosphatidylcholine (PC) and mixtures thereof. Further suitable helper lipids are e.g. 1,2-dilinoleoyl-sn-glycero-3-phosphocholine (DLPC), 1,2-dimyristoyl-sn-glycero-phosphocholine (DMPC), 1,2-dipalmitoyl-sn-glycero-3-phosphocholine (DPPC), 1,2-diundecanoyl-sn-glycero-phosphocholine (DUPC), 1-palmitoyl-2-oleoyl-sn-glycero-3-phosphocholine (POPC), 1,2-di-O-octadecenyl-sn-glycero-3-phosphocholine (18:0 Diether PC), 1-oleoyl-2-cholesterylhemisuccinoyl-sn-glycero-3-phosphocholine (OChemsPC), 1-hexadecyl-sn-glycero-3-phosphocholine (C16 Lyso PC), 1,2-dilinolenoyl-sn-glycero-3-phosphocholine, 1,2-diarachidonoyl-sn-glycero-3-phosphocholine, 1,2-didocosahexaenoyl-sn-glycero-3-phosphocholine, 1,2-diphytanoyl-sn-glycero-3-phosphoethanolamine (ME 16.0 PE), 1,2-distearoyl-sn-glycero-3-phosphoethanolamine, 1,2-dilinoleoyl-sn-glycero-3-phosphoethanolamine, 1,2-dilinolenoyl-sn-glycero-3-phosphoethanolamine, 1,2-diarachidonoyl-sn-glycero-3-phosphoethanolamine, 1,2-didocosahexaenoyl-sn-glycero-3-phosphoethanolamine, 1,2-dioleoyl-sn-glycero-3-phospho-rac-(1-glycerol) sodium salt (DOPG), sphingomyelin, and mixtures thereof.

Further suitable helper lipids and sterol lipids are e.g. disclosed in WO 2017/099823 A1 and WO 2020/061284 A1 which are incorporated herein by reference.

Certain (molar) ratios in the LNP composition have turned out to be particularly suitable for achieving improved LNPs, in particular with regard to storage stability and/or transformation efficiency:
It is thus preferred that the ether lipid fraction (as defined above) accounts for 1 mol% - 20 mol% of total lipids, preferably 2 mol% - 15 mol% of total lipids, even more preferably 4 mol% - 12 mol% of total lipids, especially 6 mol% - 10 mol% of total lipids or even 7 mol% - 9 mol% of total lipids of the LNP.

Alternatively, or in addition thereto, it is preferred that the molar ratio of ether lipid fraction to helper lipid fraction is from 20:1 to 1:20, preferably from 15:1 to 1:10, more preferably from 12:1 to 1:3, even more preferably from 8:1 to 4:1, especially from 7:1 to 5:1.

Further, alternatively, or in addition thereto, it is preferred that the molar ratio of ether lipid fraction to sterol lipid fraction is from 0.25:1 to 1:30, preferably from 0.5:1 to 1:20, more preferably from 1:1 to 1:10, even more preferably from 1:2 to 1:6, especially from 1:3 to 1:5.

Particularly good results were achieved with the following LNP, which thus forms another preferred embodiment of the invention (total lipids of the LNP, i.e. 100 mol%):
(a) 40 mol% - 70 mol% cationic lipid fraction,
(b) 5 mol% - 20 mol% helper lipid fraction,
(c) 20 mol% - 40 mol% sterol lipid fraction,
(d) 0.1 mol% - 4 mol% stabilizer fraction, and
(e) 1 mol% - 20 mol% ether lipid fraction (in particular 2 mol% - 15 mol%, even more preferably 4 mol% - 12 mol%, especially 6 mol% - 10 mol% or even 7 mol% - 9 mol%).

As evident to the skilled person upon reading the present application, these ratios and mol% given above may be observed as mean values over an entire population of LNPs (e.g. all LNPs present in a pharmaceutical composition).

The pharmaceutical composition of the present invention (which comprises a plurality of the inventive LNP) is preferably provided with at least one excipient. Excipients suitable for the pharmaceutical composition of the present invention are known to the person skilled in the art, upon having read the present specification, for example water (especially water for injection), saline, Ringer's solution, dextrose solution, buffers, Hank solution, 5% dextrose in saline, substances that enhance isotonicity and chemical stability, buffers and preservatives. This pharmaceutical composition can (as a drug) be administered via appropriate procedures known to the skilled person (upon having read the present specification) to a patient or individual in need thereof (i.e. a patient or individual having or having the risk of developing the diseases or conditions mentioned herein). The preferred route of administration of said pharmaceutical composition is parenteral administration, in particular through intraperitoneal, subcutaneous, intramuscular and/or intravenous administration. The dosage and method of administration depends on the individual patient or individual to be treated. Said pharmaceutical composition can be administered in any suitable dosage known from other biological dosage regimens or specifically evaluated and optimised for a given individual. For example, the nucleic acid cargo may be present in the pharmaceutical composition in an amount from 1 mg to 10 g, preferably 50 mg to 2 g, in particular 100 mg to 1 g. Usual dosages can also be determined on the basis of kg body weight of the patient, for example preferred dosages are in the range of 0.1 mg to 100 mg/kg body weight, especially 1 to 10 mg/kg body weight (per administration session). The administration may occur e.g. once daily, once every other day, once per week or once every two weeks. As the preferred mode of administration of the inventive pharmaceutical composition is parenteral administration, the pharmaceutical composition according to the present invention is preferably liquid or ready to be dissolved in liquid such sterile, de-ionised or distilled water or sterile isotonic phosphate-buffered saline (PBS). Preferably, 1000 µg (dry-weight) of such a composition comprises 0.1-990 µg, preferably 1-900µg, more preferably 10- 200µg compound, and option-ally 1-500 µg, preferably 1-100 µg, more preferably 5-15 µg (buffer) salts (preferably to yield an isotonic buffer in the final volume), and optionally 0.1-999.9 µg, preferably 100-999.9 µg, more preferably 200-999 µg other excipients. Preferably, 100 mg of such a dry composition is dissolved in sterile, de-ionised/distilled water or sterile isotonic phosphate-buffered saline (PBS) to yield a final volume of 0.1-100 ml, preferably 0.5-20 ml, more preferably 1-10 ml.

According to a particular preference, the LNP of the present invention has a z-average diameter between 10 nm and 900 nm, preferably between 20 nm and 750 nm, more preferably between 30 nm and 500 nm, especially between 40 nm and 250 nm or even between 50 nm and 150 nm as determined by DLS, in particular according to ISO 22412:2017. The z-average diameter as defined in ISO 22412-2017 is determined by the cumulants method and yields a scattered light intensity-weighted harmonic mean particle diameter. For instance, Markova et al, 2022, discloses in detail how to measure the z-average diameter of LNPs.

The present invention further relates to the following embodiments:
Embodiment 1. An LNP encapsulating a nucleic acid cargo, wherein the LNP comprises at least
   - a cationic lipid fraction preferably comprising at least one ionizable lipid, and
   - a stabilizer fraction;
      wherein the LNP comprises at least one GDGT lipid.
Embodiment 2. The LNP of embodiment 1, wherein the nucleic acid cargo comprises an mRNA.
Embodiment 3. The LNP of embodiment 1 or 2, wherein the stabilizer fraction comprises at least one PEG lipid.
Embodiment 4. The LNP of any one of embodiments 1 to 3, wherein the LNP further comprises a sterol lipid fraction, preferably comprising cholesterol.
Embodiment 5. The LNP of any one of embodiments 1 to 4, wherein the LNP further comprises a helper lipid fraction.
Embodiment 6. The LNP of any one of embodiments 1 to 5, wherein the at least one GDGT lipid comprises at least one caldarchaeol, preferably selected from the group consisting of unsubstituted caldarchaeol, phosphatidylinositol (PI)-caldarchaeol, dihexose(2Hex)-caldarchaeol and 2Hex-PI-caldarchaeol, hexose(Hex)-caldarchaeol, sulfono-trihexose(3Hex)-caldarchaeol, sulfono-3Hex-PI-caldarchaeol, and any mixtures thereof.
Embodiment 7. The LNP of any one of embodiments 1 to 6, wherein the LNP comprises at least one further ether lipid, preferably a diether lipid, in particular an archaeol.
Embodiment 8. The LNP of any one of embodiments 1 to 7, wherein the LNP comprises an ether lipid fraction comprising at least one GDGT lipid (preferably at least two different GDGT lipids, more preferably at least three different GDGT lipids, especially at least four different GDGD lipids) and preferably at least one further ether lipid; in particular comprising archaeols and caldarchaeols, preferably with a composition as given in Table 1 below.
Embodiment 9. The LNP of embodiment 8, wherein the ether lipid fraction comprises ether lipids obtainable by extraction from an archaeal culture, preferably a *Sulfolobus* culture, more preferably a *Sulfolobus acidocaldarius* culture.
Embodiment 10. The LNP of embodiment 9, wherein the entire ether lipid fraction is obtainable by extraction from said culture.
Embodiment 11. The LNP of any one of embodiments 1 to 10, wherein the ether lipid fraction accounts for 1 mol% - 20 mol% of total lipids, preferably 2 mol% - 15 mol% of total lipids, even more preferably 4 mol% - 12 mol% of total lipids, especially 6 mol% - 10 mol% of total lipids or even 7 mol% - 9 mol% of total lipids.
Embodiment 12. The LNP of any one of embodiments 1 to 11, wherein the molar ratio of ether lipid fraction to helper lipid fraction (in particular to DSPC, if present) is from 20:1 to 1:20, preferably from 15:1 to 1:10, more preferably from 12:1 to 1:3, even more preferably from 8:1 to 4:1, especially from 7:1 to 5:1.
Embodiment 13. The LNP of any one of embodiments 1 to 12, wherein the molar ratio of ether lipid fraction to sterol lipid fraction (in particular to cholesterol, if present) is from 0.25:1 to 1:30, preferably from 0.5:1 to 1:20, more preferably from 1:1 to 1:10, even more preferably from 1:2 to 1:6, especially from 1:3 to 1:5.
Embodiment 14. The LNP of any one of embodiments 1 to 13, wherein the total lipids of the LNP comprise (preferably consist of) :
   (a) 40 mol% - 70 mol% cationic lipid fraction,
   (b) 5 mol% - 20 mol% helper lipid fraction,
   (c) 20 mol% - 40 mol% sterol lipid fraction,
   (d) 0.1 mol% - 4 mol% stabilizer fraction, and/or
   (e) 1 mol% - 20 mol% ether lipid fraction.
Embodiment 15. The LNP of any one of embodiments 1 to 14, wherein the cationic lipid fraction comprises at least one cationic lipid (such as an ionizable lipid) selected from the group consisting of [(4-hydroxybutyl)azanediyl]di(hexane-6,1-diyl) bis(2-hexyldecanoate) (ALC-0315), heptadecan-9-yl 8-{(2-hydroxyethyl)[6-oxo-6-(undecyloxy)hexyl]amino}octanoate (SM-102),3-(didodecylamino)-N1, N1, 4-tridodecyl-1-piperazineethanamine (KL10), N1-[2-(didodecylamino)ethyl] N1,N4,N4-tridodecyl-1,4-piperazinediethanamine (KL22), 14,25-ditridecyl-15,18,21,24-tetraaza-octatriacontane (KL25), 1,2-dilinoleyloxy-N,N-dimethylaminopropane (DLin-DMA), 2,2-dilinoleyl-4-dimethylaminomethyl-[1,3]-dioxolane (DLin-K-DMA), heptatriaconta-6,9,28,31-tetraen-19-yl-4-(dimethylamino)butanoate (DLin-MC3-DMA), 2,2-dilinoleyl-4-(2 dimethylaminoethyl)-[1,3]-dioxolane (DLin-KC2-DMA), 1,2-dioleyloxy-N,N-dimethylaminopropane (DODMA), 2-({8 [(3β)-cholest-5-en-3-yloxy]octyl}oxy) N,N dimethyl-3-[(9Z,12Z)-octadeca-9,12-dien-1-yloxy]propan-1-amine (Octyl-CLinDMA), (2R)-2-({8-[(3β)-cholest-5-en-3-yloxy]octyl}oxy)-N,N-dimethyl-3-[(9Z,12Z)-octadeca-9,12-dien-1-yloxy]propan-1-amine (Octyl-CLinDMA (2R)), (2S) 2-({8-[(3β)-cholest-5-en-3-yloxy]octyl}oxy)-N,N-dimethyl-3-[(9Z,12Z)-octadeca-9,12-dien-1-yloxy]propan-1-amine (Octyl-CLinDMA (2S)) and mixtures thereof. Alternatively, or in addition thereto, the cationic lipid is preferably selected from the group consisting of (20Z,23Z)-N,N-dimethylnonacosa-20,23-dien-10-amine, (17Z,20Z)-N,N-dimemylhexacosa-17,20-dien-9-amine, (1Z,19Z)-N5N-dimethylpentacosa-16, 19-dien-8-amine, (13Z,16Z)-N,N-dimethyldocosa-13,16-dien-5-amine, (12Z,15Z)-N,N dimethylhenicosa-12,15-dien-4-amine, (14Z,17Z)-N,N-dimethyltricosa-14,17-dien-6-amine, (15Z,18Z)-N,N-dimethyltetracosa-15,18-dien-7-amine, (18Z,21Z)-N,N-dimethylheptacosa-18,21-dien-10-amine, (15Z,18Z)-N,N-dimethyltetracosa-15,18-dien-5-amine, (14Z,17Z)-N,N-dimethyltricosa-14,17-dien-4-amine, (19Z,22Z)-N,N-dimeihyloctacosa-19,22-dien-9-amine, (18Z,21 Z)-N,N-dimethylheptacosa-18,21-dien-8-amine, (17Z,20Z)-N,N-dimethylhexacosa-17,20-dien-7-amine, (16Z,19Z)-N,N-dimethylpentacosa-16,19-dien-6-amine, (22Z,25Z)-N,N-dimethylhentriaconta-22,25-dien-10-amine, (21Z,24Z)-N,N-dimethyltriaconta-21,24-dien-9-amine, (18Z)-N,N-dimetylheptacos-18-en-10-amine, (17Z)-N,N-dimethylhexacos-17-en-9-amine, (19Z,22Z)-N,N-dimethyloctacosa-19,22-dien-7-amine, N,N-dimethylheptacosan-10-amine, (20Z,23Z)-N-ethyl-N-methylnonacosa-20,23-dien-10-amine, 1-[(11Z,14Z)-1-nonylicosa-11,14-dien-1-yl]pyrrolidine, (20Z)-N,N-dimethylheptacos-20-en-10-amine, (15Z)-N,N-dimethyl eptacos-15-en-10-amine, (14Z)-N,N-dimethylnonacos-14-en-10-amine, (17Z)-N,N-dimethylnonacos-17-en-10-amine, (24Z)-N,N-dimethyltritriacont-24-en-10-amine, (20Z)-N,N-dimethylnonacos-20-en-10-amine, (22Z)-N,N-dimethylhentriacont-22-en-10-amine, (16Z)-N,N-dimethylpentacos-16-en-8-amine, (12Z,15Z)-N,N-dimethyl-2-nonylhenicosa-12,15-dien-1-amine, (13Z,16Z)-N,N-dimethyl-3-nonyldocosa-13,16-dien-1-amine, N,N-dimethyl-1-[(1S,2R)-2-octylcyclopropyl] eptadecan-8-amine, 1-[(1S,2R)-2-hexylcyclopropyl]-N,N-dimethylnonadecan-10-amine, N,N-dimethyl-1-[(1S,2R)-2-octylcyclopropyl]nonadecan-10-amine, N,N-dimethyl-21-[(1S,2R)-2-octylcyclopropyl]henicosan-10-amine,N,N-dimethyl-1-[(1S,2S)-2-{[(1R,2R)-2-pentylcyciopropyl]methyl}cyclopropyl]nonadecan-10-amine,N,N-dimethyl-1-[(1S,2R)-2-octylcyclopropyl]hexadecan-8-amine, N,N-dimethyl-[(1R,2S)-2 undecyIcyclopropyl]tetradecan-5-amine, N,N-dimethyl-3-{7-[(1S,2R)-2-octylcyclopropyl]heptyl} dodecan-1-amine, 1-[(1R,2S)-2-heptylcyclopropyl]-N,N-dimethyloctadecan-9-amine, 1-[(1S,2R)-2-decylcyclopropyl]-N,N-dimethylpentadecan-6-amine, N,N-dimethyl-1-[(1S,2R)-2-octylcyclopropyl]pentadecan-8-amine, R-N,N-dimethyl-1-[(9Z,12Z)-octadeca-9,12-dien-1-yloxy]-3-(octyloxy)propan-2-amine, S-N,N-dimethyl-1-[(9Z,12Z)-octadeca-9,12-dien-1-yloxy]-3-(octyloxy)propan-2-amine, 1-{2-[(9Z,12Z)-octadeca-9,12-dien-1-yloxy]-1-[(octyloxy)methyl]ethyl}pyrrolidine, (2 S)-N,N-dimethyl-1-[(9Z,12Z)-octadeca-9,12-dien-1-yloxy]-3-[(5Z)-oct-5-en-1-yloxy]propan-2-amine, 1-{2-[(9Z,12Z)-octadeca-9,12-dien-1-yloxy]-1-[(octyloxy)methyl]ethyl}azetidine, (2S)-1-(hexyloxy)-N,N-dimethyl-3-[(9Z,12Z)-octadeca-9,12-dien-1-yloxy]propan-2-amine, (2S)-1-(heptyloxy)-N,N-dimethyl-3-[(9Z,12Z)-octadeca-9,12-dien-1-yloxy]propan-2-amine, N,N-dimethyl-1-(nonyloxy)-3-[(9Z,12Z)-octadeca-9,12-dien-1-yloxy]propan-2-amine, N,N-dimethyl-1-[(9Z)-octadec-9-en-1-yloxy]-3-(octyloxy)propan-2-amine; (2S)-N,N-dimethyl-1-[(6Z,9Z,12Z)-octadeca-6,9,12-trien-1-yloxy]-3-(octyloxy)propan-2-amine, (2S)-1-[(11Z,14Z)-icosa-11,14-dien-1-yloxy]-N,N-dimethyl-3-(pentyloxy)propan-2-amine, (2S)-1-(hexyloxy)-3-[(11Z,14Z)-icosa-11,14-dien-1-yloxy]-N,N-dimethylpropan-2-amine, 1-[(11Z,14Z)-icosa-11,14-dien-1-yloxyl-N,N-dimethyl-3-(octyloxy)propan-2-amine, 1-[(13Z,16Z)-docosa-13,16-dien-1-yloxy]-N,N-dimethyl-3-(octyloxy)propan-2-amine, (2S)-1-[(13Z,16Z)-docosa-13,16-dien-1-yloxy]-3-(hexyloxy)-N,N-dimethylpropan-2-amine, (2S)-1-[(13Z)-docos-13-en-1-yloxy]-3-(hexyloxy)-N,N-dimethylpropan-2-amine, 1-[(13Z)-docos-13-en-1-yloxy]-N,N-dimethyl-3-(octyloxy)propan-2-amine, 1-[(9Z)-hexadec-9-en-1-yloxy]-N,N-dimethyl-3-(octyloxy)propan-2-amine, (2R)-N,N-dimethyl-H(1-metoyloctyl)oxy]-3-[(9Z,12Z)-octadeca-9,12-dien-1-yloxy]propan-2-amine, (2R)-1-[(3,7-dimethyloctyl)oxy]-N,N-dimethyl-3-[(9Z,12Z)-octadeca-9,12-dien-1-yloxy]propan-2-amine, N,N-dimethyl-1-(octyloxy)-3-({8-[(1S,2S)-2-[(1R,2R)-2-pentylcyclopropyl]methyl}cyclopropyl]octyl}oxy)propan-2-amine, N,N-dimethyl-1-{[8-(2-oclylcyclopropyl)octyl]oxy}-3-(octyloxy)propan-2-amine, and (11E,20Z,23Z)- N,N-dimethylnonacosa-11,20,2-trien-10-amine, and pharmaceutically acceptable salts and stereoisomers thereof, and mixtures thereof.
Embodiment 16. The LNP of any one of embodiments 1 to 15, wherein the helper lipid fraction comprises a helper lipid selected from the group consisting of distearoylphosphatidylcholine (DSPC), dioleoylphosphatidylethanolamine (DOPE), Dipalmitoylphosphatidylcholine (DOPC), phosphatidylcholine (PC), 1,2-dilinoleoyl-sn-glycero-3-phosphocholine (DLPC), 1,2-dimyristoyl-sn-glycero-phosphocholine (DMPC), 1,2-dipalmitoyl-sn-glycero-3-phosphocholine (DPPC), 1,2-diundecanoyl-sn-glycero-phosphocholine (DUPC), 1-palmitoyl-2-oleoyl-sn-glycero-3-phosphocholine (POPC), 1,2-di-O-octadecenyl-sn-glycero-3-phosphocholine (18:0 Diether PC), 1-oleoyl-2-cholesterylhemisuccinoyl-sn-glycero-3-phosphocholine (OChemsPC), 1-hexadecyl-sn-glycero-3-phosphocholine (C16 Lyso PC), 1,2-dilinolenoyl-sn-glycero-3-phosphocholine, 1,2-diarachidonoyl-sn-glycero-3-phosphocholine, 1,2-didocosahexaenoyl-sn-glycero-3-phosphocholine, 1,2-diphytanoyl-sn-glycero-3-phosphoethanolamine (ME 16.0 PE), 1,2-distearoyl-sn-glycero-3-phosphoethanolamine, 1,2-dilinoleoyl-sn-glycero-3-phosphoethanolamine, 1,2-dilinolenoyl-sn-glycero-3-phosphoethanolamine, 1,2-diarachidonoyl-sn-glycero-3-phosphoethanolamine, 1,2-didocosahexaenoyl-sn-glycero-3-phosphoethanolamine, 1,2-dioleoyl-sn-glycero-3-phospho-rac-(1-glycerol) sodium salt (DOPG), sphingomyelin and mixtures thereof.
Embodiment 17. A pharmaceutical composition comprising the LNP of any one of embodiments 1 to 16 and preferably at least one excipient.
Embodiment 18. The pharmaceutical composition of embodiment 17, wherein the pharmaceutical composition is a vaccine, preferably an mRNA vaccine.
Embodiment 19. The pharmaceutical composition of embodiment 17 or 18, for use in prevention or treatment of a disease or condition in a patient.
Embodiment 20. The LNP or pharmaceutical composition of any one of embodiments 1 to 19, wherein the LNP has a z-average diameter between 10 nm and 900 nm, preferably between 20 nm and 750 nm, more preferably between 30 nm and 500 nm, especially between 40 nm and 250 nm or even between 50 nm and 150 nm (as determined by DLS, in particular according to ISO 22412:2017).

The present invention is further illustrated by the following figures and examples, without being restricted thereto.
**Fig. 1****:** GDGTs increased cellular uptake of LNPs by a factor of 30 compared to a reference LNP formulation used in an EMA-approved SARS-CoV-2 mRNA vaccine. (a) Partially replacing the helper lipid DSPC in the LNP formulation with GDGTs ("TELs") lead to an increased rhodamine-lipid fluorescence signal in the cells upon incubation with the LNPs. Reference had DSPC at a concentration of 9.6 mol% total lipids. (b) Partially replacing cholesterol ("Chol") in the LNP formulation with GDGTs ("TELs") lead to an increased rhodamine-lipid fluorescence signal in the cells upon incubation with the LNPs. Reference had cholesterol at a concentration of 42.6 mol% total lipids. (c) Repetition of experiments with two references ("A" and "B") confirmed that partially replacing the helper lipid DSPC in the LNP formulation with GDGTs ("TELs") lead to an increased rhodamine-lipid fluorescence signal in the cells upon incubation with the LNPs, with optimal results achieved at about 8 mol% GDGTs ("TELs").
**Fig. 2****:** GDGTs increased transfection efficiency of LNPs by a factor of 90 compared to a reference LNP formulation used in an EMA-approved SARS-CoV-2 mRNA vaccine. Partially replacing the helper lipid DSPC in the LNP formulation with GDGTs ("TELs") lead to an increased EGFP mRNA expression in the cells upon incubation with the LNPs (which encapsulated the EGFP mRNA), as measured by fluorescence (arbitrary units, y axis). Reference had DSPC at a concentration of 9.6 mol% total lipids.

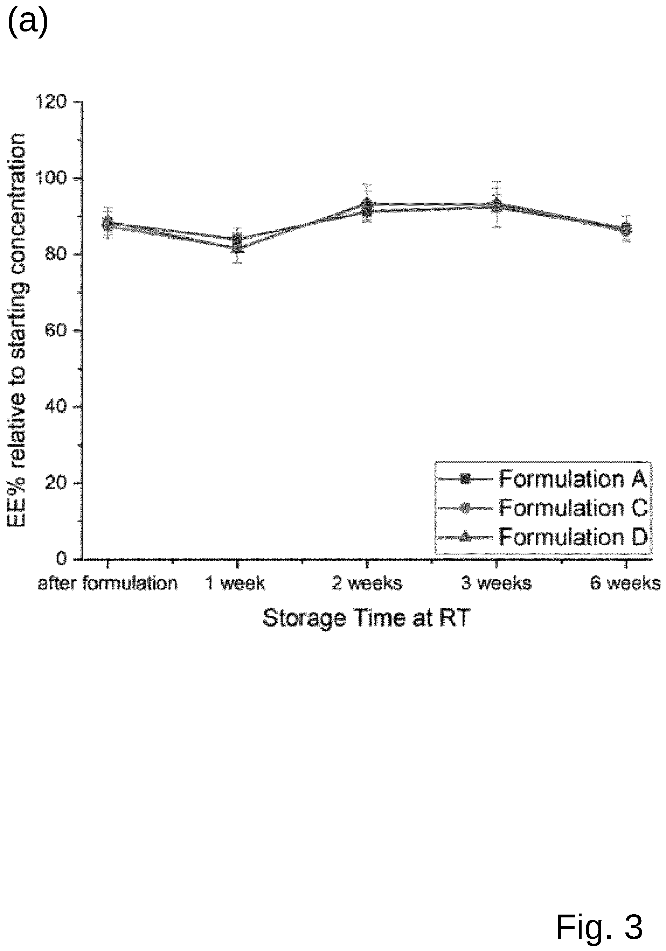

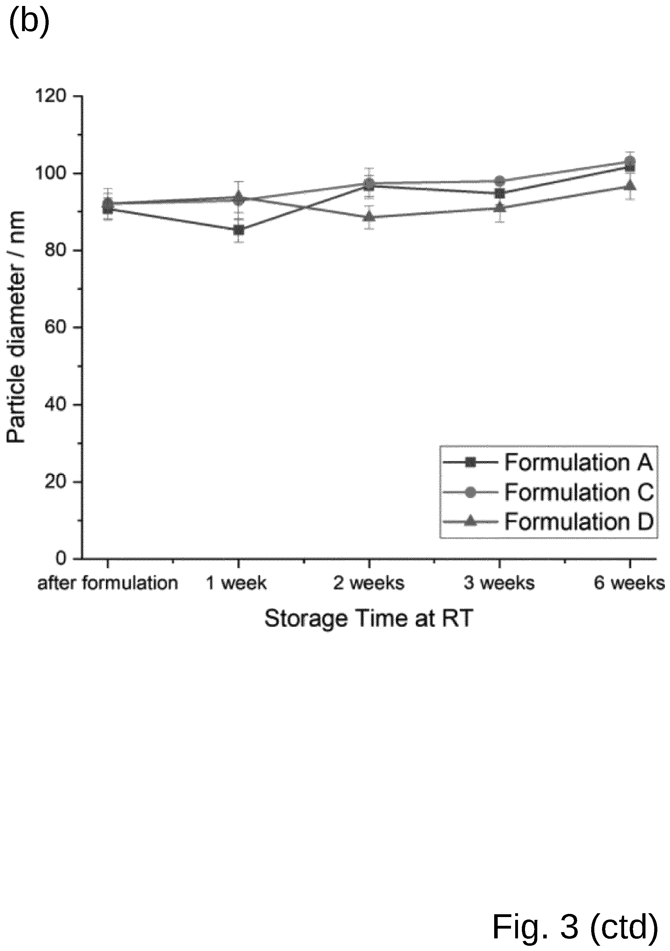

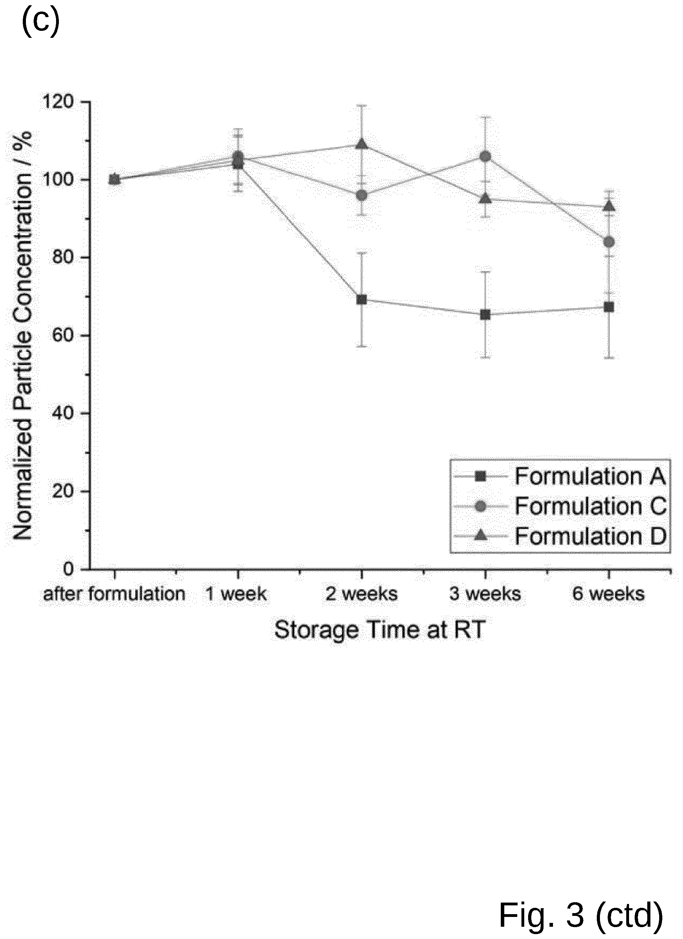

**Fig. 3****:** GDGT-LNPs were stable at room temperature (RT, 25°C). For the three test formulations A, C and D, (a) encapsulation efficiency (EE), (b) particle diameter and (c) particle concentration remained comparatively constant over time, indicating excellent storage stability.

### Example

### Formulation of LNPs

Lipid stock solutions (archaeal membrane lipids obtained from *Sulfolobus acidocaldarius* including GDGTs (see Table 1 below; see also WO 2020/187526 A1), DSPC, ALC-0315, ALC-0159, cholesterol, rhodamine) were filtered through a 0.2 µm polytetrafluoroethylene (PTFE) filter and mixed according to the molar ratio used in the respective experiment (see also Tables 2 and 3 below). The organic solvent for the reference formulation was ethanol while as in formulations containing GDGTs, a mixture of dimethyl sulfoxide (DMSO) and 2-propanol (2:3) served as the organic solvent. The aqueous phase was prepared dissolving Poly(A) (Carl Roth) or mRNA coding for EGFP (CleanCap, 5moU), respectively, in a 10 mM citrate buffer (pH=4.0). LNPs were formulated using a NanoAssembleR Ignite (Precision Nanosystems) at a total flow rate of 12 mL/min and a flow rate ratio of 3:1 (aqueous phase:organic phase). Immediately after formulation, LNPs were diluted 1:2 in a 10 mM PBS (pH=7.4). Removal of organic solvent was achieved via dialysis (SpectraPor, 6-8 kDa) against 10 mM phosphate-buffered saline (PBS) buffer (pH=7.4). Physiochemical characterization of the LNPs was performed via the Ribogreen assay (mRNA content) and Zetasizer analysis (size, polydispersity index (PDI), Zeta potential), see Table 4 below. Zetasizer is available from Malvern Panalytical Ltd, UK.

A typical lipid composition for the GDGT-containing archaeal membrane lipid stock solution (i.e. the ether lipid fraction) is given in Table 1:

| Lipid component | Relative abundance within the ether lipid fraction (mol%) |
|---|---|
| archaeols | 5-25% |
| unsubstituted caldarchaeols | 15-50% |
| Hex-caldarchaeols | 8-14% |
| PI-caldarchaeols | 3-10% |
| 2Hex-caldarchaeols | 10-22% |
| 2Hex-PI-caldarchaeols | 3-10% |
| sulfono-3Hex-PI-caldarchaeols | 0-5% |

**Table 2 gives tested variations in helper lipid composition:**

| LNP sample | DSPC (mol%) | GDGTs (mol%) | Cholesterol (mol%) |
|---|---|---|---|
| *reference* | 9.4 | 0.0 | 42.6 |
| 1 | 1.4 | 8.0 | 42.6 |
| 3 | 5.4 | 4.0 | 42.6 |
| 5 | 7.4 | 2.0 | 42.6 |

**Table 3 gives tested variations in cholesterol lipid composition:**

| LNP sample | DSPC (mol%) | GDGTs (mol%) | Cholesterol (mol%) |
|---|---|---|---|
| *reference* | 9.4 | 0.0 | 42.6 |
| 2 | 9.4 | 8.0 | 34.6 |
| 4 | 9.4 | 4.0 | 38.6 |
| 6 | 9.4 | 2.0 | 40.6 |

**Table 4 shows characteristics of tested LNPs (EE: encapsulation efficiency), the z-average diameter was determined by DLS:**

| LNP sample | EE (%) | PDI | Z-average (nm) |
|---|---|---|---|
| *reference* | 81.2 ± 3.8 | 0.17 | 60.4 ± 1.5 |
| 1 | 86.4 ± 2.7 | 0.12 | 75.8 ± 0.8 |
| 3 | 84.5 ± 3.9 | 0.13 | 65.6 ± 0.9 |
| 5 | 86.9 ± 6.2 | 0.09 | 59.9 ±0.9 |
| 2 | 89.7 ± 2.8 | 0.11 | 63.1 ± 1.1 |
| 4 | 86.7 ± 5.5 | 0.15 | 63.7 ± 0.8 |
| 6 | 82.4 ± 2.2 | 0.13 | 58.3 ± 1.3 |

A high encapsulation efficiency and a low PDI (i.e. monodisperse distribution) were observed for the LNP samples.

### Uptake of LNPs

After two passages, Caco-2 cells were seeded in 48 well plates at a cell density of 10⁵ cells/well in 1 mL medium (Dulbecco's Modified Eagle Medium (DMEM) + L-glutamine, 10% fetal bovine serum (FBS), 1% antibiotic mix). After 24 hours of incubation (37°C, 5% CO₂), LNPs were added to the wells at a cargo concentration of 5.2 µg Poly(A)/well. After another incubation for 24 hours, cells were washed twice with 500 µL 10 mM PBS buffer in order to remove LNPs remaining in the supernatant. This was followed by the addition of Hoechst solution and another incubation step of 5 min. After two washing steps with 500 µL 10 mM PBS buffer, the cellular uptake of LNPs (excitation/emission=570 nm/590 nm) and the number of cell nuclei (excitation/emission=460 nm/ 490 nm) were analyzed with confocal microscopy (Olympus IX83).

GDGTs increased cellular uptake of LNPs compared to a reference LNP formulation used in an EMA-approved SARS-CoV-2 mRNA vaccine (see Fig. 1).

### Transfection using LNPs

After two passages, Caco-2 cells were seeded in 48 well plates at a cell density of 10⁵ cells/well in 950 µL medium (DMEM + L-glutamine, 10% FBS, 1% antibiotic mix). After 24 hours of incubation (37°C, 5% CO₂), 500 µL of depleted medium were replaced with new medium. Subsequently, LNPs were added to the wells at a concentration of 6.4 µg mRNA coding for EGFP /well. Cells were incubated for 60 hours and fluorescence intensity (excitation/emission=488 nm/ 509 nm) was measured after 17, 24, 36, 48, 60 hours using confocal microscopy (Olympus IX83).

GDGTs increased transfection efficiency of LNPs compared to a reference LNP formulation used in an EMA-approved SARS-CoV-2 mRNA vaccine (see Fig. 2).

### Storage of LNPs

After formulation, LNPs were stored in 2 mL Eppendorf tubes at 4°C and 25°C (room temperature), respectively. Physiochemical characterization via Ribogreen assay and ZetaSizer analysis was conducted in certain time intervals over a period of 6 weeks.

GDGT-LNPs turned out to be stable even at room temperature (see Fig. 3).

### Non-patent references

Aldosari, Basmah N., et al. "Lipid nanoparticles as delivery systems for RNA-based vaccines." Pharmaceutics 13.2 (2021): 206.
Daswani, Varsha P., et al. "The Polar Lipid Fraction E from Sulfolobus acidocaldarius Can Be Used as Liposomal Drug Stabilizing Agents to Reduce the Leakage of the Antivascular Drug Combretastatin A4 Disodium Phosphate from Tetraether/Diester Hybrid Archaeosomes." Biophysica 1.4 (2021): 474-486.
Hou, Xucheng, et al. "Lipid nanoparticles for mRNA delivery." Nature Reviews Materials 6.12 (2021): 1078-1094.
Jain, Samta, et al. "Biosynthesis of archaeal membrane ether lipids." Frontiers in microbiology 5 (2014): 641.
Kaur, Gurmeet, et al. "Archaeosomes: an excellent carrier for drug and cell delivery." Drug delivery 23.7 (2016): 2497-2512.
Markova, Natalia, et al. "Biophysical Characterization of Viral and Lipid-Based Vectors for Vaccines and Therapeutics with Light Scattering and Calorimetric Techniques." Vaccines 10.1 (2021): 49.
Midoux, Patrick, et al. "Lipid-based mRNA vaccine delivery systems." Expert review of vaccines 14.2 (2015): 221-234.
Patel, Girishchandra B., et al. "Archaeobacterial ether lipid liposomes (archaeosomes) as novel vaccine and drug delivery systems." Critical reviews in biotechnology 19.4 (1999): 317-357.
Paunovska, Kalina, et al. "Drug delivery systems for RNA therapeutics." Nature Reviews Genetics 23.5 (2022): 265-280.
Schouten, Stefan, et al. "The organic geochemistry of glycerol dialkyl glycerol tetraether lipids: A review." Organic geochemistry 54 (2013): 19-61.
Siliakus, Melvin F., et al. "Adaptations of archaeal and bacterial membranes to variations in temperature, pH and pressure." Extremophiles 21.4 (2017): 651-670.
Szoka Jr, Frank, et al. "Comparative properties and methods of preparation of lipid vesicles (liposomes)." Annual review of biophysics and bioengineering 9.1 (1980): 467-508.
van Hoogevest, Peter. "Review-an update on the use of oral phospholipid excipients." European journal of pharmaceutical sciences 108 (2017): 1-12.
Vishwakarma, Nikhar, et al. "Lipid-based nanocarriers for lymphatic transportation." AAPS PharmSciTech 20.2 (2019): 1-13.

## Claims

1. A lipid nanoparticle (LNP) encapsulating a nucleic acid cargo, wherein the LNP comprises at least
- a cationic lipid fraction, and
- a stabilizer fraction;
wherein the LNP comprises at least one glycerol dialkyl glycerol tetraether (GDGT) lipid.

2. The LNP of claim 1, wherein the nucleic acid cargo comprises a messenger ribonucleic acid (mRNA).

3. The LNP of claim 1 or 2, wherein the stabilizer fraction comprises at least one polyethylenglycol (PEG) lipid.

4. The LNP of any one of claims 1 to 3, wherein the LNP further comprises a sterol lipid fraction, preferably comprising cholesterol.

5. The LNP of any one of claims 1 to 4, wherein the LNP further comprises a helper lipid fraction; preferably wherein the helper lipid fraction comprises a helper lipid selected from the group consisting of distearoylphosphatidylcholine (DSPC), dioleoylphosphatidylethanolamine (DOPE), Dipalmitoylphosphatidylcholine (DOPC), phosphatidylcholine (PC), 1,2-dilinoleoyl-sn-glycero-3-phosphocholine (DLPC), 1,2-dimyristoyl-sn-glycero-phosphocholine (DMPC), 1,2-dipalmitoyl-sn-glycero-3-phosphocholine (DPPC), 1,2-diundecanoyl-sn-glycero-phosphocholine (DUPC), 1-palmitoyl-2-oleoyl-sn-glycero-3-phosphocholine (POPC), and mixtures thereof.

6. The LNP of any one of claims 1 to 5, wherein the at least one GDGT lipid comprises at least one caldarchaeol, preferably selected from the group consisting of unsubstituted caldarchaeol, phosphatidylinositol (PI)-caldarchaeol, dihexose(2Hex)-caldarchaeol and 2Hex-PI-caldarchaeol, hexose(Hex)-caldarchaeol, sulfono-trihexose(3Hex)-caldarchaeol, sulfono-3Hex-PI-caldarchaeol, and any mixtures thereof.

7. The LNP of any one of claims 1 to 6, wherein the LNP comprises at least one further ether lipid, preferably a diether lipid, in particular an archaeol.

8. The LNP of any one of claims 1 to 7, wherein the LNP comprises an ether lipid fraction comprising at least one GDGT lipid and at least one further ether lipid; preferably wherein the ether lipid fraction comprises archaeols and caldarchaeols.

9. The LNP of claim 8, wherein the ether lipid fraction comprises ether lipids obtainable by extraction from an archaeal culture, preferably a *Sulfolobus* culture, more preferably a *Sulfolobus acidocaldarius* culture.

10. The LNP of claim 9, wherein the entire ether lipid fraction is obtainable by extraction from said culture.

11. The LNP of any one of claims 1 to 10, wherein the ether lipid fraction accounts for 1 mol% - 20 mol% of total lipids, preferably 2 mol% - 15 mol% of total lipids, even more preferably 4 mol% - 12 mol% of total lipids, especially 6 mol% - 10 mol% of total lipids or even 7 mol% - 9 mol% of total lipids.

12. The LNP of any one of claims 1 to 11, wherein the molar ratio of ether lipid fraction to helper lipid fraction is from 20:1 to 1:20, preferably from 15:1 to 1:10, more preferably from 12:1 to 1:3, even more preferably from 8:1 to 4:1, especially from 7:1 to 5:1.

13. The LNP of any one of claims 1 to 12, wherein the molar ratio of ether lipid fraction to sterol lipid fraction is from 0.25:1 to 1:30, preferably from 0.5:1 to 1:20, more preferably from 1:1 to 1:10, even more preferably from 1:2 to 1:6, especially from 1:3 to 1:5.

14. The LNP of any one of claims 1 to 13, wherein the total lipids of the LNP comprise:
(a) 40 mol% - 70 mol% cationic lipid fraction,
(b) 5 mol% - 20 mol% helper lipid fraction,
(c) 20 mol% - 40 mol% sterol lipid fraction,
(d) 0.1 mol% - 4 mol% stabilizer fraction, and
(e) 1 mol% - 20 mol% ether lipid fraction.

15. A pharmaceutical composition comprising the LNP of any one of claims 1 to 14, preferably wherein the pharmaceutical composition is a vaccine.
